# EUROPÄISCHE PATENTANMELDUNG

(11) **EP 1 006 127 A2**
(43) Veröffentlichungstag der Anmeldung: **07.06.2000**
(21) Anmeldenummer: 99123643.1
(22) Anmeldetag: 27.11.1999
(51) Int. Cl.: C07K 17/00, C08G 69/10, B01J 31/06, C07D 301/19

(54) **Katalysatoren für die enantioselektive Epoxidierung von Kohlenstoff-Kohlenstoff Doppelbindungen**

(30) Priorität: 03.12.1998 DE 19855859
(71) Anmelder: Degussa-Hüls Aktiengesellschaft, 60287 Frankfurt am Main (DE)
(72) Erfinder: Drauz, Karlheinz, Prof. Dr., 63579 Freigericht (DE); Roberts, Stan M., Prof. Dr., Parlegate, Neston 646RN (GB); Geller, Thomas, Dr., 51373 Leverkusen (DE); Dhanda, Anupma, 3000 Helsingoer (DK)

(57) **Zusammenfassung**

Diastereomerenangereicherte und enantiomerenangereicherte Homopolyaminosäure, welche adsorptiv auf einem unlöslichen Trägermaterial gebunden sind, eignen sich zur einfachen Epoxidierung von C=C-Doppelbindungen.

Verfahren und Verwendung.

## Beschreibung

Die vorliegende Erfindung richtet sich auf neue trägergebundene, diastereomerenangereicherte und enantiomerenangereicherte Homopolyaminosäuren, ein Verfahren zu deren Herstellung, deren Verwendung in einem Verfahren zur enantioselektiven Epoxidierung von C=C-Doppelbindungen und bevorzugte Zwischenprodukte.

Enantioselektive Epoxidierungsreaktionen sind wichtige Reaktionen zum Aufbau chiraler Intermediate für die organische Synthese. Insbesondere die asymmetrische Epoxidierung von Allylalkoholen nach Sharpless et al. und die mangansalenvermittelte enantioselektive Epoxidierung nach Jacobsen et al. sind in der organischen Synthese zum Aufbau chiraler Moleküle wohl etabliert (Sharpless et al., J. Am. Chem. Soc. 1980, 102, 5974; J. Am. Chem. Soc. 1987, 109, 5765; J. Org. Chem. 1986, 51, 1922; Jacobsen et al., J. Am. Chem. Soc. 1990, 112, 2801; J. Am. Chem. Soc. 1991, 113, 7063).

Eine andere Möglichkeit zur asymmetrischen Epoxidation von C=C-Doppelbindungen wurde bei der Reaktion von Chalconen mit Wasserstoffperoxid in Gegenwart von enantiomerenangereicherten Polyaminosäuren entdeckt (Colonna et al., Org. Synth.; Mod. Trends, Proc. IUPAC Symp. 6th, 1986, 275; Julia et al., Angew. Chem., Int. Ed. Engl., 1980, 19, 929).

Die eben vorgestellten Synthesemethoden haben allesamt den Nachteil, daß sie auf ein relativ eng begrenztes Substratspektrum anwendbar sind. Aus dieser Tatsache und der fortwährenden Forschungstätigkeit auf diesem Gebiet ist die Notwendigkeit abzuleiten, weiterhin verbesserte Epoxidierungsprozedere zu finden.

Im Stande der Technik sind bis dato zwei unterschiedliche Varianten der Julia-Colonna-Epoxidierungsreaktion - die zweiphasige bzw. dreiphasige Variante - bekannt (S. M. Roberts et al. Chem. Commun. 1998, 1159; WO 96/33183). Die zweiphasige Variante bedient sich eines organischen Lösungsmittels und arbeitet mit in diesen Lösungsmitteln löslichen Oxidantien in Gegenwart der unlöslichen Homopolyaminosäuren. Die dreiphasige Variante benutzt neben dem wasserunlöslichen organischen Lösungsmittel ebenso Wasser als 3. Phase. So können vorteilhafterweise wasserlösliche Oxidantien für die Reaktion - ggf. in Gegenwart von Phasentransferkatalysatoren - herangezogen werden.

Aus den die letztgenannte Epoxidierungsreaktion betreffenden Schriften wird jedoch deutlich, daß entscheidende Mängel dieser Epoxidierungsmethoden im Hinblick auf deren Anwendung in einem industriellen Prozeß die geringen Raum/Zeit-Ausbeuten (Reaktionszeiten im Bereich von Tagen) und die mitunter für manche Substrate schlechten ee-Werte sind.

Auf der anderen Seite hat man gefunden, daß der Einsatz von Immobilisierungstechniken für enzymvermittelte Reaktionen im Hinblick auf z. B. die Rückgewinnbarkeit des Katalysators und Steigerung der optischen und chemischen Ausbeute von Vorteil sein kann (EP 0 799 894 A2 sowie Tetrahedron Asymmetry 1991, 2, 931).

Angesichts des hierin angegebenen und diskutierten Standes der Technik war es mithin Aufgabe der Erfindung trägergebundene, diastereomerenangereicherte und enantiomerenangereichterte Homopolyaminosäuren anzugeben, welche es gestatten, C=C-Doppelbindungen in Gegenwart eines Oxidans zu epoxidieren. Dabei sollte im Vergleich mit den trägerungebundenen Katalysatoren des Standes der Technik insbesondere eine höhere Reaktionsgeschwindigkeit gepaart mit einer besseren chiralen Induktion gegeben sein. Noch eine Aufgabe der Erfindung ist darin zu sehen, die trägergebundenen Katalysatoren möglichst einfach und damit kostengünstig herstellen zu können sowie eine bessere Handhabbarkeit im Hinblick auf die Wiedergewinnung des Katalysators nach der Reaktion zu gewährleisten, was für deren Anwendung im technischen Maßstab Vorteile bringt.

Diese und nicht näher genannte weitere sich aus dem Stand der Technik in naheliegender Weise ergebende Aufgaben werden durch Homopolyaminosäuren des Anspruchs 1 gelöst. Bevorzugte Ausgestaltungen sind Gegenstand der Ansprüche 2 bis 6. Anspruch 7 gibt ein äußerst bevorzugtes und einfaches Verfahren zur Herstellung der erfindungsgemäßen Katalysatoren an, Ansprüche 8 bis 11 schützen bevorzugte Verwendungen. Anspruch 12 ist auf ein bevorzugtes Zwischenprodukt gerichtet, Ansprüche 13 bis 15 betreffen bevorzugte Ausgestaltungen des erfindungsgemäßen Zwischenprodukts. Anspruch 16 ist auf ein Verfahren zur Herstellung des Zwischenprodukts gerichtet, während die Ansprüche 17 und 18 deren Verwendung schützen.

Dadurch, daß diastereomerenangereicherte und enantiomerenangereicherte Homopolyaminosäure adsorptiv auf einem unlöslichen Trägermaterial gebunden sind, erhält man Katalysatoren für die enantioselektive Epoxidierung, welche äußerst einfach und damit kostengünstig herzustellen und darüber hinaus völlig überraschend die Reaktionsgeschwindigkeiten bei dieser Reaktion gegenüber den Katalysatoren des Standes der Technik beträchtlich heraufzusetzen im Stande sind. Gleichzeitig wird in nicht vorhersehbarer Weise eine Steigerung der Ausbeute und der Enantiomerenüberschüsse bei den Epoxidprodukten erhalten. Darüber hinaus können die erfindungsgemäßen trägergebundenen Homopolyaminosäuren sehr gut recycliert werden und sind aufgrund ihrer Vergrößerung und Heterogenisierung gerade im technischen Maßstab äußerst gut handhabbare Katalysatoren.

Zur Herstellung der trägergebundenen Katalysatoren können verschiedene diastereomeren- und enantiomerenangereicherte Homopolyaminosäuren eingesetzt werden. Vorzugsweise werden allerdings Homopolyaminosäuren aus der Gruppe Polyneopentylglycin, Polyleucin, Polyisoleucin, Polyvalin und Polyalanin sowie Polyphenylalanin benutzt. Aus dieser Gruppe ist Polyneopentylglycin am meisten zu bevorzugen.

Die Kettenlänge der Polyaminosäuren ist so zu wählen, daß einerseits die chirale Induktion bei der Reaktion nicht beeinträchtigt wird und andererseits die Kosten zur Synthese der Polyaminosäuren nicht zu groß werden. Vorzugsweise liegt die Kettenlänge der Homopolyaminosäuren zwischen 5 und 100, vorzugsweise 7 bis 50, Aminosäuren. Ganz besonders bevorzugt ist eine Kettenlänge von 10 bis 40 Aminosäuren.

Bevorzugt ist weiterhin die Ausführungsform, in der die Homopolyaminosäuren mit polyfunktionellen Aminen vernetzt sind bzw. durch andere organische Polymere vergrößert vorliegen. Vorteilhafterweise setzt man als Vernetzer Amine, wie z. B. 1,3-Diaminopropan, Propylenimintetraamindendrimere der 1. Generation oder quervernetztes Hydroxy- oder Aminopolystyrol ein. Als Polymervergrößerer kommt bevorzugt polyethylenglykolpolystyrolbasierende Nucleophile in Frage. Derart veränderte Polyaminosäuren sind in Chem. Commun 1998, 841f, 1159f und Tetrahedron Asymmetry 1997, 8, S. 3165f erwähnt.

Die unlöslichen Trägermaterialien sind solche, welche vorzugsweise auf Siliziumoxid-Basis aufgebaut sind, wie z. B. Molsieb, Silicagel oder Zeolithe sowie Celite 521® oder Celite Hyflo Super Cell®, Wessalith® Day P. Vorteilhaft sind auch Silicagele mit definierten Porengrößen wie z. B. CPC I oder CPC II. Bevorzugt sind auch Zuckerderivate wie Nitrocellulose, Cellulose oder Aktivkohle als Trägermaterial.

Das Verhältnis von Trägermaterial zu Polyaminosäure ist durch zwei Grenzen gegeben. Einerseits kann nur eine bestimmte Anzahl von Polyaminosäure auf dem unlöslichen Träger adsorbiert werden, auf der anderen Seite läßt die chirale Induktion ab weniger als 10 Gew.-% von Polyaminosäure zu Träger nach. Bevorzugt liegt das Verhältnis von Homopolyaminosäure zu Trägermaterial zwischen 1 : 7 und 2 : 1 Gew.-teile, besonders bevorzugt zwischen 1 : 1 und 1 : 4 Gew.-teile.

Gegenstand der Erfindung ist ebenfalls ein einfaches aber äußerst vorteilhaftes Verfahren zur Herstellung der Homopolyaminosäuren, welches sich dadurch auszeichnet, daß man eine Mischung aus Homopolyaminosäure und Trägermaterial in einem organischen Lösungsmittel suspendiert und anschließend den Rückstand nach Filtration trocknet.

Ein weiterer Aspekt der vorliegenden Erfindung ist die Verwendung der trägergebundenen Homopolyaminosäuren in einem Verfahren zur enantioselektiven Epoxidierung von C=C-Doppelbindungen. Weiterhin sind die erfindungsgemäßen Katalysatoren bevorzugt in Verfahren einzusetzen, welche in einer Vorrichtung durchgeführt werde, die befähigt ist, nur den Katalysator zurückzuhalten. Vorzugsweise handelt es sich bei dieser Vorrichtung um einen Enzym-Membran-Reaktor (C. Wandrey in Enzymes as Catalysts in Organic Synthesis, Ed.: M. Schneider, Dordrecht Riedel 1986, 263-284). Ebenso bevorzugt ist als Vorrichtung auch ein einfacher Festbettreaktor, wie z. B. eine Chromatographiesäule.

Ein nächster Aspekt der Erfindung beschäftigt sich mit Polyneopentylglycin, welches vorzugsweise diastereomerenrein und enantiomerenrein vorliegt.

Durch die Bereitstellung von Polyneopentylglycin als trägerungebundenem Katalysator für die enantioselektive Epoxidierung von C=C-Doppelbindungen enthaltenden Substraten wird es in nicht vorhersehbarer Weise ermöglicht, die chirale Induktion bei der Epoxidierungsreaktion gegenüber den Homopolyaminosäuren des Standes der Technik (PLL hat sich als bester Katalysator für diese Reaktion durchgesetzt und ist kommerziell erhältlich) wesentlich zu verbessern. Darüber hinaus wurde völlig überraschend festgestellt, daß bei verkürzter Reaktionszeit eine oftmals guantitativere Ausbeute erhalten wird.

Bezüglich der Kettenlänge, Vernetzung und Polymervergrößerung sei auf das oben zu den Homopolyaminosäuren dargestellte verwiesen.

Polyneopentylglycin wird vorzugsweise aus den N-Carbonsäureanhydriden (NCAS) von Neopentylglycin durch nucleophile initiierende Polymerisation analog der im Stand der Technik bekannten Homopolyaminosäuren hergestellt.

Polyneopentylglycin kann in einem Verfahren zur Herstellung von enantiomerenangereicherten Epoxiden Verwendung finden. Darüber hinaus ist es ganz besonders bevorzugt Homopolyneopentylglycin in einem Verfahren zur Herstellung von trägergebundenen Epoxidierungskatalysatoren der vorliegenden Erfindung zu benutzen.

Wie erwähnt können die bei der Epoxidierung einzusetzenden Homopolyaminosäuren nach Methoden des Standes der Technik hergestellt werden (siehe z. B. in Flisak et al., J. Org. Chem. 1993, 58, 6247). Die Methode ist auf beide optischen Antipoden der Aminosäuren anzuwenden. Der Einsatz einer bestimmten Antipode einer Polyaminosäure korreliert mit der Stereochemie des Epoxids, d. h. eine Poly-L-aminosäure führt zur optischen Antipode des Epoxids, das mit einer Poly-D-aminosäure erhalten wird.

Es hat sich gezeigt, daß eine Behandlung der Homopolyaminosäure vor dessen Einsatz mit basisch wäßrigen Medien, wie in der EP 0 403 252 A2 beschrieben, die Reaktionsgeschwindigkeit in der Epoxidierungsreaktion weiter absinken läßt.

Die Adsorption der Homopolyaminosäure auf dem Trägermaterial erfolgt vorteilhafterweise durch 48 h Rühren in einem organischen Lösungsmittel, wie z. B. in Ethern wie THF, in Gegenwart des Trägermaterials. Anschließend wird der Feststoff abfiltriert und getrocknet.

Bei der vorliegenden erfindungsgemäßen Reaktion geht man im allgemeinen so vor, daß man die zu verwendende Homopolyaminosäure bzw. die abgeänderten Derivate, wie vernetzte, polymervergrößerte oder auf unlöslichen Trägern adsorbierte Homopolyaminosäure, in dem einzusetzenden Reaktionslösungsmittelgemisch suspendiert, anschließend das Oxidans hinzufügt, ggf. den pH-Wert einstellt und abschließend das Substrat der allgemeinen Formel I zufügt. Die Einhaltung der Reihenfolge der Arbeitsschritte ist nicht zwingend, doch sollte das Oxidans vorzugsweise als letztes zur Mischung hinzugefügt werden, um eine ungewollte nichtinduzierte Epoxidierung zu Beginn der Reaktion, wenn noch kein Katalysator beigemengt wurde, nicht zuzulassen, da dieses zu geringeren ee-Werten im Produkt Anlaß gibt. Die Verfahren sind im Stand der Technik beschrieben (S. M. Roberts et al., Tetrahedron: Asymmetry 1997, 8, 3163-3173, J. Chem. Soc., Perkin Trans. 1, 1996, 4, 343-348; Chem. Commun. 1998, 1159-1160 sowie dort zitierte Literatur; WO 96/33183; EP 0 403 252). Die Anwendung des Polyneopentylglycins oder der trägergebundenen Katalysatoren gemäß der vorliegenden Erfindung erfolgt einfach durch Austausch der in den beschriebenen Verfahren benutzten Polyaminosäuren durch den neuen Katalysator. Es sei angemerkt, daß die erfindungsgemäßen Katalysatoren sowohl mit der zweiphasigen wie mit der dreiphasigen Variante gute Ergebnisse liefern. Für die trägergebundenen Homopolyaminosäuren ist im Rahmen der Erfindung jedoch die zweiphasige Variante bevorzugt.

Die Überlegenheit der trägergebundenen Katalysatoren gegenüber trägerungebundenen wird durch folgendes Schema 1 verdeutlicht.

### Schema 1: (siehe Beispiel 1.a)

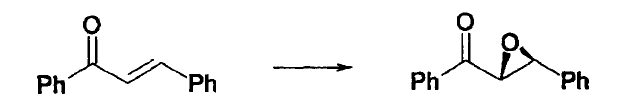

| Nr. | Trägermaterial | t [min] | Umsetzung [%] | ee-Wert [%] |
|---|---|---|---|---|
| 1 | - | 120 | 43 | 93 |
| 2 | Silicagel | 30 | 99 | 95 |
| 3 | CPC I | 35 | 99 | 97 |
| 4 | CPC II | 30 | 76 | 95 |
| 5 | Molekularsieb | 120 | 93 | 95 |

Einen Einfluß auf die Güte der Epoxidierung hat die Beladung des Trägermaterials mit Homopolyaminosäuren (Schema 2).

### Schema 2: (siehe Beispiel 1.b)

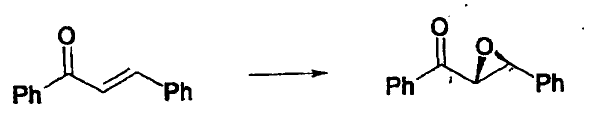

| Nr. | PLL/Silicagelverhältnis | t [min] | Umsetzung [%] | ee-Wert [%] |
|---|---|---|---|---|
| 1 | 1:1 | 30 | 97 | 94 |
| 2 | 1:3,4 | 30 | 99 | 95 |
| 3 | 1:7 | 210 | 72 | 93 |
| 4 | 1:10 | 210 | 47 | 93 |

Weitere Beispiele, die Vorteile der trägergebundenen Homopolyaminosauren wiedergeben sind im folgenden dargestellt (Schema 3).

### Schema 3: (ziehe Beispiel 1.c)

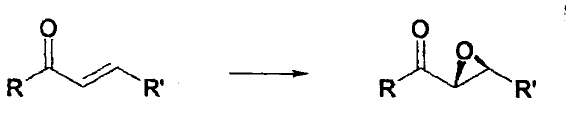

| Nr. | Kat. | t [h] | Umsetzung [%] | ee-Wert [%] | R,R' |
|---|---|---|---|---|---|
| 1 | PLL | 24 | 50 | 93 | o-NH₂-Ph, Ph |
| 2 | PLL-Träger | 3 | 85 | 93 | dto. |
| 3 | PLL | 26 | 56 | 89 | iPr, Ph |
| 4 | PLL-Träger | 14 | 78 | 93 | dto. |

Die Reaktionsgemische werden nach Verfahren, welche dem Fachmann bekannt sind, aufgearbeitet. Das lösliche Epoxid wird vorteilhafterweise durch Filtration von dem Katalysator getrennt und anschließend wäßrig aufgearbeitet. Falls erwünscht, kann eine abschließende Chromatographie des Epoxids an Kieselgel zur Reinigung erfolgen.

Die Filtrierbarkeit der trägergebundenen Katalysatoren ist gegenüber den trägerungebundenen deutlich verbessert. So liegen die Homopolyaminosäuren als Pasten vor, welche den Filter verkleben, wohingegen die trägergebundenen von fester Natur sind und mit einfachen Filtriergerät abgetrennt werden können.

Dies macht sich vor allem bei der Wiederverwendung der erfindungsgemäßen trägergebundenen Katalysatoren positiv bemerkbar. Der Verlust an Katalysatormenge bei wiederholtem Einsatz (Nr gibt die Anzahl der Einsätze an) ist wesentlich verringert gegenüber den trägerungebundenen (Schema 6).

### Schema 6: (siehe Beispiel 1.d)

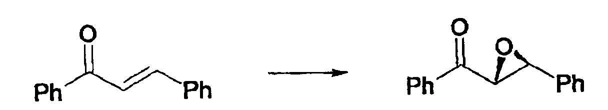

| Nr | PLL-trägergeb. | | | | PLL-trägerungeb. | | | |
|---|---|---|---|---|---|---|---|---|
| 1 | t [min] | Ausb. [%] | ee[%] | Kat.-verlust [%] | t [min] | Ausb. [%] | ee[%] | Kat.-verlust [%] |
| 2 | 200 | 96 | 93 | 3 | 60 | 48 | 97 | 10 |
| 3 | 115 | 90 | 95 | 3 | 60 | 82 | 97 | 7 |
| 4 | 95 | 94 | 95 | 7 | 60 | 81 | 98 | 8 |
| 5 | 105 | 97 | 98 | 2 | 60 | 79 | 97 | 4 |
| 6 | 100 | 91 | 95 | 1 | 150 | 82 | 97 | 8 |
| 7 | 110 | 95 | 98 | 2 | >240 | 80 | 96 | 15 |

Aus den oben dargelegten Ergebnissen zeigt sich der Vorteil den der Einsatz der tragergebundenen gegenüber den trägerungebundenen Homopolyaminosäuren im Hinblick auf Aktivität, Selektivität, Handling und Recyclingfähigkeit besitzt. Dies wird allein durch die einfache Adsorption der Homopolyaminosäuren auf unlöslichen Trägermaterialien erreicht, was äußerst überraschend dennoch aber um so vorteilhafter erscheint.

Untenstehend sind Ergebnisse der Epoxidierungsreaktionen von PLL-CLAMPS verglichen mit PLN-CLAMPS dargestellt. PLL bedeutet Poly-L-leucin, PLN Poly-L-neopentylglycin. Alle Ergebnisse wurden per chiraler HPLC gemessen (siehe Beispiel 2).

### Schema 7:

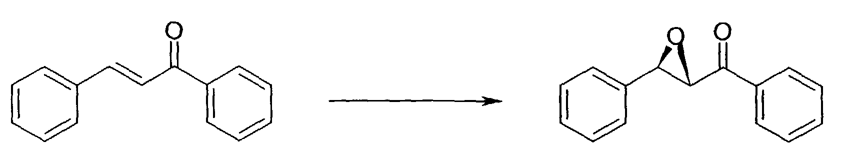

| Bedingungen | Zeit [h] | Umsetzung (%) | ee-Wert (%) |
|---|---|---|---|
| 2-phasig/PLL | 18 | 90 | 95 |
| 2-phasig/PLN | 6 | 98-100 | >95 |
| 3-phasig/PLL | 0.5 | 90 | 95 |
| 3-phasig/PLN | 0.5 | 90 | 96 |

### Schema 8:

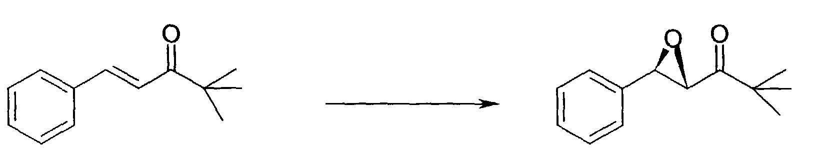

| Bedingungen | Zeit [h] | Umsetzung (%) | ee-Wert (%) |
|---|---|---|---|
| 2-phasig/PLL | 22 | 95 | 94 |
| 2-phasig/PLN | 3 | 90 | 98 |

### Schema 9:

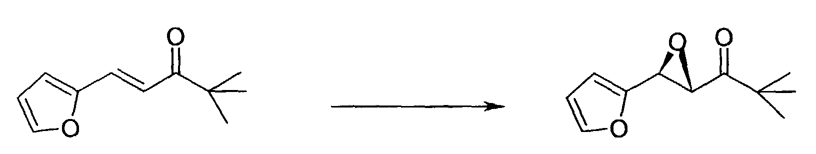

| Bedingungen | Zeit [h] | Umsetzung (%) | ee-Wert (%) |
|---|---|---|---|
| 2-phasig/PLL | 8 | 83 | 95 |
| 2-phasig/PLN | 2 | 93 | >95 |

### Schema 10:

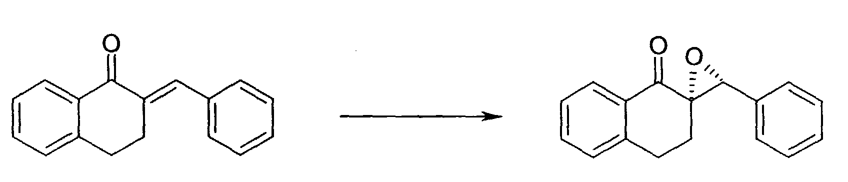

| Bedingungen | Zeit [h] | Umsetzung (%) | ee-Wert (%) |
|---|---|---|---|
| 2-phasig/PLL | 32 | 60 | 60 |
| 2-phasig/PLN | 7 | 80 | 75 |

Wie aus diesen Beispielen deutlich hervorgeht, besitzt Poly-L-neopentylglycin ebenfalls deutliche Vorteile gegenüber den im Stande der Technik bekannten Homopolyaminosäuren im Hinblick auf Aktivität und Selektivität.

Mit den hier vorgestellten Homopolyaminosäuren und modifizierten Derivaten werden somit zum einen bessere ee-Werte bei der Epoxidierung erhalten und zum anderen können die Reaktionszeiten bei dieser Transformation deutlich erniedrigt werden. Dies war so aus dem Stand der Technik nicht ohne weiteres herleitbar. Zwar ist es - wie oben angegegeben - bekannt, Enzyme auf unlösliche Träger zu absorbieren, doch wird dadurch in keinster Weise nahegelgt, daß die erfindungsgemäßen Verbindungen durch eine solche Maßnahme die beobachteten Verbesserungen erfahren. Im Gegenteil hätte der Fachmann erwarten dürfen, daß mit der Adsorption des Polymers auf dem festen Trägermaterial eine konformative Umorientierung und Fixierung dieser Konformation dergestalt eintritt, daß dadurch deren chirale Induktion beeinträchtigt werden würde. Da der Mechanismus der vorgestellten Epoxidierungsreaktion bis heute nicht bekannt ist, war es daher äußerst überraschend, daß die eben beschriebene Fixierung zu einer Erhöhung der Aktivität und Induktion beiträgt und nicht umgekehrt.

Darüber hinaus wird durch die Möglichkeit, Enzyme auf unlöslichen Trägern zu binden, in keinster Weise nahegelegt, daß die hier betrachteten Homopolyaminosäuren auf den unlöslichen Trägermaterialien entsprechend fest zu adsorbieren sind. Enzyme sind Makromoleküle, welche ganz andere nichtkovalente Wechselwirkungen mit Substraten eingehen können, verglichen mit den Homopolyaminosäuren dieser Erfindung.

Gegenüber den chemisch modifizierten, polymervergrößerten Homopolyaminosäuren ist die Herstellung der trägergebundenen Homopolyaminosäuren durch physikalische Adsorption derart simpel und damit kostengünstig, daß deren Anwendung im technischen Maßstab bevorzugt ist. Weiterhin ist von Vorteil, daß die Handhabbarkeit und Wiedergewinnbarkeit gegenüber den trägerungebundenen Homopolyaminosäuren deutlich erhöht ist, da durch die Heterogenisierung eine bessere Filtrierbarkeit gegeben ist. Normale Homopolyaminosäuren des Standes der Technik sind pastös und befähigt, Poren der Filter und Membranen zu verstopfen, was gerade für deren technische Anwendung beträchtliche Schwierigkeiten bereiten würde. Damit sind die erfindungsgemäßen Epoxidierungskatalysatoren prädestiniert für den technischen Einsatz in vorteilhaften Apparaturen wie Festbettreaktor und Enzym-Membran-Reaktor (C. Wandrey in Enzymes as Catalysts in Organic Synthesis, Ed.: M. Schneider, Dordrecht Riedel 1986, 263-284), welche eine für einen industriellen Prozeß äußerst bevorzugte kontinuierliche bzw. guasikontinuierliche Fahrweise der Reaktion gestatten.

Mithin ist diese Erfindung daher angetan, die Anwendung der erfindungsgemäßen Katalysatoren in einem industriellen Prozeß ökonomisch gesehen erst vorteilhaft erscheinen zu lassen.

Unter dem Begriff Homopolyaminosäuren versteht der Fachmann Polymere aus Aminosäuren einer Provenienz. Im Rahmen der Erfindung können die eingesetzten Homopolyaminosäuren jedoch auch Copolymere verschiedener Aminosäuren aufgebaut sein, bei der jedoch Domänen, welche die chirale Induktion bedingen, aus einheitlichen Aminosäuren bestehen sollten. Mithin bezieht sich der Ausdruck Homopolyaminosäuren auch auf aus heterochiralen Aminosäuren aufgebaute Polymere. Wiederum gilt, daß die Domänen, welche die chirale Induktion bedingen, aus einer stereochemisch einheitlichen Folge von Aminosäuren bestehen sollten.

Unter trägergebunden wird die physikalische Adsorption von Molekülen auf den erfindungsgemäßen Trägermaterialien verstanden.

Unter dem Begriff enantiomerenangereichert ist im Rahmen der Erfindung der Anteil eines Enantiomers im Gemisch mit seiner optischen Antipode in einem Bereich von >50 % und <100 % zu verstehen.

Unter dem Begriff diastereomerenangereichert wird im Rahmen der Erfindung der Anteil eines Diastereomeren im Gemisch mit seinen anderen Diastereomeren in einem Bereich von >50 % und <100 % verstanden.

### Beispiele:

### 1) Verwendung von trägergebundenem PLL als Katalysator

Standardbedingungen für die Testung der Katalysatoren (gelten für alle trägergebundenen Katalysatoren soweit nichts anders bestimmt ist).

Katalysatorherstellung: 70 mg PLL (1,3-Diaminopropan als Initiator für die Polymerisation benutzt, Chem. Commun. 1998, 1159; S.M. Roberts et al. J. Chem. Soc. Perkin Trans. I, 1998, 3171) wird mit 240 mg Trägermaterial in 2 ml THF für 48h mittels Magnetrührer gerührt. Anschließend filtriert man und trocknet den Rückstand 1h bei 1,1 kPa und RT, dann bei 50-55°C und 0.008 kPa für 7h.

Epoxidierungsreaktion: 18 mg *trans*-Chalcon, 10 mg UHP (Harnstoffperoxidverbindung), 155 mg trägergebundener Katalysator und 15 µl DBU werden in 1 ml THF bei RT gerührt. Die Umsetzung und der ee-Wert werden mit chiraler HPLC bestimmt.

HPLC-Bedingungen: Die chirale HPLC wurde an Chiralpak AD-Säulen durchgeführt. Dazu wurde ein Aliquot der Reaktionsmischung entnommen und nach Filtration bei 1,00 ml/min Flußrate mit 10 Gew.-% EtOH/Hexan als Laufmittel bei 254 nm analysiert.

### 1.a) Verschiedene Träger

Silicagel = Silicagel 60 (230 - 400 mesh, Merck)
CPC I = controlled poresize carrier (375 Å, 30 - 45 mesh, Fluka)
CPC II = controlled poresize carrier silane coated, abgeleitet vom 3-Aminopropylrest (375 Å, 30 - 45 mesh, Fluka),
Celithe 521® (Aldrich) , Celithe Hyflo Super Cell® (Fluka), Wessalith® Day P. = SiO₂,
Molsieb (SiO₂, Lot: MPM TI, Degussa)
Zeolith TS1 (5,5 Å, 3% Ti)

Reaktionsbedingungen für PLL-Einsatz:
Analog Literaturmethoden - zweiphasige Variante

Umsetzungen und ee-Werte wurden per chiraler HPLC bestimmt.

Retentionszeiten der enantiomeren Epoxide: 15,9 min (Hauptenantiomer) und 23,7 min

Unter Eintrag 1 wurden Trägermaterialien und Homopolyaminosäure erst kurz vor der Epoxidierung vereinigt.

### 1.b) Unterschiedliche Beladung

Trägergebundene Katalysatoren mit den entsprechenden Polyaminosäure-Träger-verhältnissen wurden ansonsten gemäß Standardprozeß hergestellt. Als Trägermaterial diente Silicagel.

Umsetzung und ee-Wert wurden per chiraler HPLC detektiert. Retentionszeiten siehe Beispiel 1.a).

### 1.c) Variation der Substrate

Trägermaterial: Silicagel; Verhältnis 1:3,4

Reaktionsbedingungen für den PLL-Vergleich:
Analog Literaturmethoden - zweiphasige Variante

Einträge 1 und 2:

Umsetzung und ee-Wert wurden per chiraler HPLC 14% EtOH in Hexan, 230 nm detektiert.

Retentionszeiten der enantiomeren Epoxide: 25,6 min (Hauptenantiomer) und 31,2 min

Einträge 3 und 4:

Umsetzung und ee-Wert wurden per chiraler HPLC 5% EtOH in Hexan, 230 nm, Flußrate 0,7 ml/min detektiert.

Retentionszeiten der enantiomeren Epoxide: 12,6 min (Hauptenantiomer) und 23,3 min

### 1.d) Recyclierung des Katalysators

Reaktionsbedingungen für den PLL-Vergleich:
Analog Literaturmethoden - zweiphasige Variante
(Startmenge an Katalysator 2,0 g)

Wiedergewinnung des Katalysators:
Nach der Reaktion wurde filtriert, der Filterrückstand mit Wasser/Aceton (1:1), Aceton und EtOAc gewaschen und anschließend im Oilpumpenvakuum getrocknet.

Reaktionsbedingungen für den Einsatz des trägergebundenen Katalysators:
Trägermaterial: Silicagel; Verhältnis 1:3,4

116 mg trans-Chalcon, 65 mg UHP, 100 µl DBU wurden in 6,5 ml THF bei RT mit der entsprechenden Menge an trägergebundenem Katalysator umgesetzt (Startmenge 1,0 g).

Wiedergewinnung des Katalysators:
Nach der Reaktion wurde filtriert, der Filterrückstand mit THF, EtOH und abschließend wieder mit THF gewaschen.
Anschließend wurde der Rückstand nach Standardbedingungen getrocknet.

### 2) Verwendung von Polyneopentylglycin (PLN) als Katalysator:

Die chirale HPLC wurde an Chiralpak AD-Säulen durchgeführt. Dazu wurde ein Aliquot der Reaktionsmischung entnommen und nach Filtration bei 1,00 ml/min Flußrate analysiert.

Dabei wurden folgende Ergebnisse bzgl. der untersuchten Substrate gefunden:

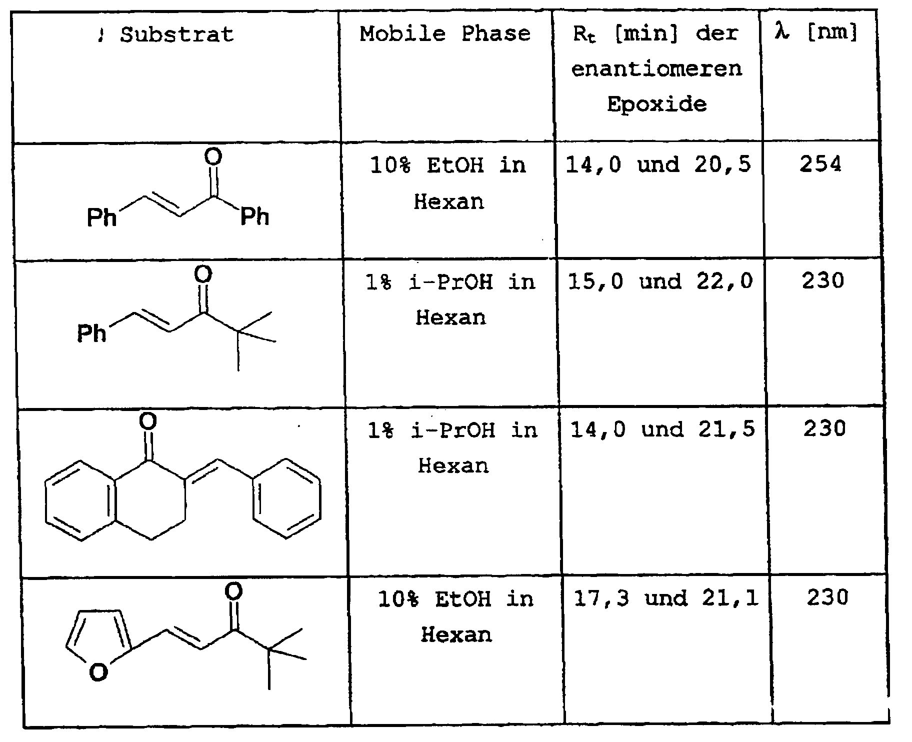

### Herstellung des Katalysators (PLN-CLAMPS):

Ein getrockneter 2 l 3-Halskolben wird mit 0.1400 mol Neopentylglycin (kauflich erhältlich) beladen und 24 h bei 100°C unter Vakuum gehalten. Anschließend wird der Kolben mit Stickstoff gespült und mit 1 l trockenem THF versetzt. Nach Erwärmen der Suspension auf ca. 50°C wird 0.0513 mol Triphosgen tropfenweise über eine Zeitraum von 15 min. hinzugegeben. Nach 2 h wird die Lösung auf RT abgekühlt, filtriert und im Vakuum eingeengt. Der kristalline Rückstand wird in einer minimalen Menge THF gelöst und durch Zugabe von 1 l n-Hexan precipitiert. Man erhält in 85%iger Ausbeute das NCA von L-Neopentylglycin als einen farblosen Feststoff vom Schmp. 129.3-131.2°C; δ_{H} (300 MHz; CDCl₃) 1.02(9H, s, 3 x CH₃), 1.65 (1H, dd, *J* 14.7 and 9.9, β-CH₂), 2.0 (1H, dd, *J* 14.7 and 2.4, β-CH₂), 4.38 (1H, dd, *J* 9.9 and 2.4, α-CH), 6.8 (1H, br, NH).

0.1180 mol L-Neopentylglycin-NCA wird in 250 ml THF mit (3.668 mmol) CLAMPS (cross-linked-amino-polystyrene) versetzt und die Mischung wurde 5 Tage bei RT unter Stickstoff gerührt. Die Suspension wird anschließend filtriert und das Filtrat wird für 30 min mit Wasser behandelt, anschließend mit Aceton/Wasser (1:1), Aceton/Wasser (4:1), Aceton (2x), EE (2x), Diethylether (2x) gespült und abschließend im Vakuum getrocknet.

Anschließend wird das Polymer in einer Mischung aus Toluol/4 M Natriumhydroxidlösung (2.5:1) für mehrere Stunden aktiviert.

PLL-CLAMPS können analog hergestellt werden (siehe auch Chem. Commun. 1998, 1159).

### Epoxidierung:

### - 3-phasige Variante

100 mg des Polymers (PLL-Clamps oder PLN-Clamps) werden in 0.8 ml Toluol und 0.2 ml 4M NaOH (12 eq.) bei 0°C im Eisbad mit 0.2 ml 30%iger wäßriger H₂O₂ (21 eq.) versetzt und 6 h bei dieser Temp. belassen. Das Enon (1 eq.) nochmals 0.5 ml H₂O₂ und 0.2 ml Toluol werden zu der Mischung gegeben. Nach verschiedenen Zeiten werden Proben entnommen und per chiraler HPLC analysiert.

### - 2-phasige Variante

100 mg des Polymers (PLL-Clamps oder PLN-Clamps) und 0.24 mmol des Enons werden in 0.8 ml THF mit 0.36 mmol (1.5 eq.) DBU und 0.28 mmol UHP (Harnstoff-Wasserstoffperoxid-Verbindung) versetzt. Man rührt die Mischung bei RT. Nach verschiedenen Zeiten werden Proben entnommen und per chiraler HPLC analysiert.

## Patentansprüche

1. Diastereomerenangereicherte und enantiomerenangereicherte Homopolyaminosäure, welche adsorptiv auf einem unlöslichen Trägermaterial gebunden ist.

2. Homopolyaminosäuren nach Anspruch 1 aus der Gruppe Polyneopentylglycin, Polyleucin, Polyisoleucin Polyvalin, Polyphenylalanin und Polyalanin.

3. Homopolyaminosäure nach Anspruch 1 und/oder 2
dadurch gekennzeichnet,
daß die Homopolyaminosäure eine Kettenlänge von 5 bis 100 Aminosäuren, vorzugsweise 7 bis 50, besitzt.

4. Homopolyaminosäure nach einem oder mehreren der Ansprüche 1 bis 3,
dadurch gekennzeichnet,
daß die Homopolyaminosäuren miteinander vernetzt vorliegen oder durch organische Polymere vergrößert sind.

5. Trägermaterial nach Anspruch 1 aus der Gruppe siliziumoxidhaltiger Verbindungen, Nitrocellulose, Cellulose oder Aktivkohle.

6. Trägermaterial nach Anspruch 5,
dadurch gekennzeichnet,
daß das Verhältnis von Homopolyaminosäure und Trägermaterial zwischen 1:7 und 2:1 Gew.-teile, vorzugsweise zwischen 1:1 und 1:4 Gew.-teile, liegt.

7. Verfahren zur Herstellung der trägergebundenen Homopolyaminosäuren nach Anspruch 1,
dadurch gekennzeichnet,
daß man die Homopolyaminosäure mit dem Trägermaterial in einem organischen Lösungsmittel suspendiert und anschließend nach Filtration den Rückstand trocknet.

8. Verwendung der Homopolyaminosäuren nach Anspruch 1 in einem Verfahren zur enantioselektiven Epoxidierung von C=C-Doppelbindungen.

9. Verwendung der Homopolyaminosäuren nach Anspruch 8,
dadurch gekennzeichnet,
daß das Verfahren in einer Vorrichtung durchgeführt wird, die befähigt ist, nur den Katalysator gemäß Anspruch 1 zurückzuhalten.

10. Verwendung der Homopolyaminosäuren nach Anspruch 9,
dadurch gekennzeichnet,
daß die Vorrichtung ein Enzym-Membran-Reaktor ist.

11. Verwendung der Homopolyaminosäuren nach Anspruch 9,
dadurch gekennzeichnet,
daß die Vorrichtung ein Festbettreaktor ist.

12. Diastereomerenangereichertes und enantiomerenangereichertes Polyneopentylglycin.

13. Polyneopentylglycin nach Anspruch 12,
dadurch gekennzeichnet,
daß es eine Kettenlänge von 5 bis 100, vorzugsweise 7 bis 50, Aminosäuren besitzt.

14. Polyneopentylglycin nach einem oder mehreren der Ansprüche 12 bis 13,
dadurch gekennzeichnet,
daß es mittels mehrfunktioneller Verbindungen vernetzt vorliegt.

15. Polyneopentylglycin nach einem oder mehreren der Ansprüche 12 bis 14,
dadurch gekennzeichnet,
daß es mit organischen Polymeren vergrößert ist.

16. Verfahren zur Herstellung von Polyneopentylglycin,
dadurch gekennzeichnet,
daß man es aus N-Carbonsäureanhydriden von Neopentylglycin gewinnt.

17. Verwendung von Polyneopentylglycin in einem Verfahren zur Herstellung von enantiomerenangereicherten Epoxiden.

18. Verwendung von Polyneopentylglycin in einem Verfahren zur Herstellung von trägergebundenen Epoxidierungskatalysatoren gemäß Anspruch 1.
